# EUROPEAN PATENT SPECIFICATION

(11) **EP 1 671 095 B1**
(45) Date of publication and mention of the grant of the patent: **15.04.2015**
(21) Application number: 04786569.6
(22) Date of filing: 25.08.2004
(51) Int. Cl.: A61B 6/00, G01N 1/00

(54) **LOCALIZATION OF A TARGET USING IN VIVO MARKERS**
LOKALISIERUNG EINES ZIELS MIT IN-VIVO-MARKERN
LOCALISATION D'UNE CIBLE AU MOYEN DE MARQUEURS IN VIVO

(30) Priority: 16.09.2003 US 664213
(43) Date of publication of application: 21.06.2006
(73) Proprietor: Varian Medical Systems, Inc., Palo Alto, CA 94304 (US)
(72) Inventor: MOSTAFAVI, Hassan, Los Altos, CA 94024 (US); PARTAIN, Larry, D., Los Altos, CA 94022 (US); SLOUTSKY, Alexander, Burlingame, CA 94010 (US); MUNRO, Peter, Mountain View, CA 94043 (US)
(74) Representative: Manley, Nicholas Michael
(86) International application number: PCT/US2004/027596
(87) International publication number: WO 2005/036124

(56) References cited:
- EP-A1- 0 427 358
- WO-A-03/025621
- WO-A1-90/11721
- WO-A1-98/18523
- US-A- 4 578 806
- US-A- 5 769 861
- US-A- 5 810 007
- US-A1- 2002 065 461
- US-A1- 2002 193 685

## Description

### TECHNICAL FIELD

This invention relates to the field of medical devices and procedures and, in particular, to localization of targets within a body.

### BACKGROUND

Radiation therapy involves medical procedures that selectively expose a target volume of a human (or animal) body, such as a cancerous tumor, to high doses of radiation. The intent of the radiation therapy is to irradiate the targeted biological tissue such that the harmful tissue is destroyed. To minimize damage to surrounding body tissues, many conventional treatment methods utilize "dose fractionating" to deliver the radiation dosage in a planned series of treatment sessions that each delivers only a portion of the total planned dosage. Healthy body tissues typically have greater capacity to recover from the damage caused by exposed radiation. Spreading the delivered radiation over many treatment sessions allows the healthy tissue an opportunity to recover from radiation damage, thus reducing the amount of permanent damage to healthy tissues while maintaining enough radiation exposure to destroy the tumor.

It is known that daily setup variation and various types of organ movement contribute to uncertainty in the position of the target relative to the treatment beam. The image quality of electronic portal imagers are now such that they can be used to produce one or more low-dose images before or during beam delivery solely for the purpose of accurate patient setup and patient position monitoring.

The conventional 2D X-ray projection images allow visualization of hard tissue such as bony anatomy. However, many soft tissue targets of radiation therapy are difficult or impossible to visualize in such images. An example is the boundaries of the prostate gland that may not be seen even in diagnostic quality 2D X-ray images. Therefore, both kilo volt (KV) and mega volt (MV) 2D imaging linear accelerators usually rely on bony anatomy for patient positioning thus resulting in inaccurate positioning of a soft tissue target, such as prostate, when it moves relative to the bony anatomy.

Even for some bony anatomy targets, e.g., spine (vertebral body) as a target of intensity modulated radio-surgery (IMRS), it is required that in addition to its position the orientation of the target be known accurately. However, because of the generally round boundaries of these targets, it is difficult to accurately estimate their pose using triangulation based on stereo pairs of 2D X-ray images.

One proposed solution is the use of computerized tomography (CT) volumetric X-ray imaging in radiation treatment rooms. Examples are CT-on-rail and cone beam CT using on-board imaging (OBI) or portal imaging. The boundaries of some soft tissue targets are more visible in CT slices. They can be contoured in the collection of slices that span the target volume thus delineating the target in 3D in much the same way as is done for treatment planning with CT images. To be clinically effective, the in-room CT as an online imaging modality requires both fast volumetric image reconstruction and fast CT contouring capability. Even if fast and reliable 3D contouring becomes available, the in-room CT equipment entails added cost and, in the case of CT-on rail, inhibiting space requirements for some clinics.

Published International Patent application WO03/025621 (RADQUAL LLC, application No. PCT/US02/30032) describes a multi-modality imaging method in which a spot marker used as a positioning aid in the imaging is placed on the patient's skin, e.g. at a recognizable spot such as the navel. First and second images of the patient are produced. The spot marker has first and second "detectable substances", the first of which appears in the first image and the second of which appears in the second image. The two images are aligned using the images of the first and second substances.

United State patent 5769861 (Vilsmeier) describes a method of localizing an instrument relative to three-dimensional corporeal data. Markers are secured to skeletal bone to define a reference system. It is suggested that scanning be carried out using computer tomography and nuclear magnetic resonance imaging, with the resulting data being combined to obtain three dimensional corporeal data.

Published International Patent Application WO 90/11721 (Lome Linda University Medical Center) discloses a patient alignment system and a procedure for radiation treatment in which a patient is immobilised within a "formfit patient pod". Reference radiographs are prepared with an x-ray system and used for repositioning the patient within the pod on subsequent occasions. CT scan data of a tissue volume of interest is obtained while the patient remains in the pod. The CT scan data is used to prepare a treatment plan. The treatment plan includes identification of an isocenter within the tissue volume at which the beam is to be directed from selected angles.

Published International Patent Application WO 98/18523 A1 discloses such a pateint positioning method whereby a two-dimensional offset between the center of the beam and a target isocenter is obtained from the positions of anatomical landmarks in master-prescription and treatment-position two-dimensional images so that the position of the patient is adjusted accordingly.

### SUMMARY OF AN EMBODIMENT OF THE DISCLOSURE

In accordance with a first aspect of the present disclosure there is a method, characterised by:
imaging a plurality of markers in a first imaging modality and determining first coordinates of the plurality of markers;
imaging the plurality of markers in a second imaging modality and determining second coordinates of the plurality of markers;
the plurality of markers being implanted markers internal to and within a body of a patient and in soft tissue within the body of the patient;
the determination of the first coordinates of the plurality of markers being made relative to a first isocenter;
the determination of the second coordinates of the plurality of markers being made relative to a second isocenter; wherein the first imaging modality is one of a MRI, an ultrasound, and an x-ray imaging modality, the first isocenter being a planned isocenter determined using an image from the first imaging modality, the second imaging modality being an x-ray imaging modality arranged to provide an image of the patient at a treatment system, the second isocenter being the treatment isocenter of the treatment beam;
determining an offset between the first coordinates and the second coordinates for at least one of the plurality of markers; and
determining a change in position of a treatment beam isocenter of a treatment beam of a treatment system relative to the target and relative to the markers; and based on the offset determined between the first coordinates and the second coordinates.

In accordance with a second aspect of the present disclosure there is an apparatus, characterised by:
means for imaging a plurality of markers in a first imaging modality;
means for imaging the plurality of markers in a second imaging modality;
the plurality of markers being implanted markers residing internal to a patient within the patient's body and in soft tissue within the body of the patient, and wherein the apparatus further comprises
means for determining first coordinates of the plurality of markers relative to a first isocenter;
means for determining second coordinates of the plurality of markers relative to a second isocenter, wherein the first imaging modality is one of a MRI, an ultrasound, and an x-ray imaging modality, the first isocenter is a planned isocenter determined using an image from the first imaging modality, the second imaging modality is an x-ray imaging modality arranged to provide an image of the patient at a treatment system, the second isocenter being the treatment isocenter of the treatment beam; the apparatus further comprises
means for determining an offset between the first coordinates and the second coordinates for at least one of the plurality of markers; and the apparatus further comprises
means for determining a change in position of a treatment beam isocenter of a treatment beam of a treatment system relative to the target and relative to the markers; and based on the offset determined between the first coordinates and the second coordinates.

The invention is as defined in the appended claims.

Additional features and advantages of the present invention will be apparent from the accompanying drawings, and from the detailed description that follows below.

### BRIEF DESCRIPTION OF THE DRAWINGS

The present invention is illustrated by way of example, and not by way of limitation, in the figures of the accompanying drawings.
Figure 1A illustrates an enlarged imaged prostrate area of patient's body having a sensor device and a plurality of marker seeds.
Figure 1B illustrates an enlarged imaged prostate area using a second imaging modality having an array of markers that are imagable and a sensor device that is not imagable.
Figure 2A illustrates one embodiment of a sensor device having markers disposed thereon.
Figure 2B illustrates an alternative embodiment of a sensor device having markers disposed therein.
Figure 3 illustrates one embodiment of a sensor device having a casing with multiple imaging properties.
Figure 4 illustrates one embodiment of an imaging system.
Figure 5 illustrates one embodiment of digital processing system of Figure 4.
Figure 6 illustrates one embodiment of a localization method.
Figure 7 illustrates one embodiment of detecting a marker and removing a false marker in an image.
Figure 8A illustrates one embodiment of a positional offset between internal markers imaged at different times.
Figure 8B illustrates one embodiment of a pair of stereo images and an epipolar line.
Figure 9 illustrates one embodiment of a median filtering of an image segment containing a marker.
Figure 10A illustrates one embodiment of an image region of interest, containing a marker, after the use of a median filter.
Figure 10B illustrates one embodiment of an image region of interest, not containing a marker, after the use of a median filter.
Figure 11A illustrates one embodiment of an image region of interest, containing a marker, after a connected component analysis with a low threshold.
Figure 11B illustrates one embodiment of an image region of interest, not containing a marker, after a connected component analysis with a low threshold.
Figure 12A illustrates an image region of interest, containing a marker, after a connected component analysis with a higher threshold than used for the image of Figure 11A.
Figure 12B illustrates an image region of interest, not containing a marker, after a connected component analysis with a higher threshold than used for the image of Figure 11B.
Figure 13 is a table illustrating the relationship between various localization parameters.
Figure 14 illustrates an alternative embodiment of localizing markers using digitally reconstructed radiographs produced from different view angles using a CT set.
Figure 15 illustrates one embodiment of graphically displaying 3D coordinates of imaged markers.

### DETAILED DESCRIPTION

In the following description, numerous specific details are set forth such as examples of specific systems, components, methods, etc. in order to provide a thorough understanding of the present invention. It will be apparent, however, to one skilled in the art that these specific details need not be employed to practice the present invention. In other instances, well-known components or methods have not been described in detail in order to avoid unnecessarily obscuring the present invention.

The present invention includes various steps, which will be described below. The steps of the present invention may be performed by hardware components or may be embodied in machine-executable instructions, which may be used to cause a general-purpose or special-purpose processor programmed with the instructions to perform the steps. Alternatively, the steps may be performed by a combination of hardware and software.

The present invention may be provided as a computer program product, or software, that may include a machine-readable medium having stored thereon instructions, which may be used to program a computer system (or other electronic devices) to perform a process according to this present invention. A machine-readable medium includes any mechanism for storing or transmitting information in a form (e.g., software, processing application) readable by a machine (e.g., a computer). The machine-readable medium may include, but is not limited to, magnetic storage medium (e.g., floppy diskette); optical storage medium (e.g., CD-ROM); magneto-optical storage medium; read-only memory (ROM); random-access memory (RAM); erasable programmable memory (e.g., EPROM and EEPROM); flash memory; electrical, optical, acoustical, or other form of propagated signal (e.g., carrier waves, infrared signals, digital signals, etc.); or other type of medium suitable for storing electronic instructions.

The present invention may also be practiced in distributed computing environments where the machine-readable medium is stored on and/or executed by more than one computer system. In addition, the information transferred between computer systems may either be pulled or pushed across the communication medium connecting the computer systems, such as in a remote diagnosis or monitoring system. In remote diagnosis or monitoring, a user may utilize the present invention to diagnose or monitor a patient despite the existence of a physical separation between the user and the patient.

A method and apparatus for localization of a sensor device and/or a target within a body using in vivo markers is discussed. In one embodiment, the target may be an anatomical landmark and the markers may be marker seeds. The markers may be implanted in the body in a target volume. The markers (e.g., radio-opaque) may be localized relative to a treatment isocenter (e.g., as part of the planning process) using an imaging technique, for examples, the CT dataset used for planning the treatment, radiographic images from a simulator, or radiographic images from the first day of treatment. The localized markers operate as a 3D reference position. Then, in a subsequent treatment session, the markers can be localized again using images (e.g., X-ray) acquired in that subsequent session. The subsequent images may be acquired using either the same imaging modality as the earlier acquired images or a different imaging modality if the markers are capable of being imaged using the different imaging modalities.

By comparing the position of the markers with their reference position, any necessary adjustments to the patient position and orientation (and/or treatment beam direction and shape) may be determined. The adjustments may be determined so that the target geometry relative to the treatment beam is as close as possible to the planned geometry.

In one embodiment, the target may be a sensor device. Although, the following discussion may be in reference to a sensor device, the sensor device may also have telemetric capabilities such as a responder or a transponder. In one embodiment, the method and apparatus described provides a means to localize in the body one or more sensor devices (e.g., sensor, responder, transponder, etc.). The sensor device may be situated in the body through various means, for example, implantation through injection. The site may be, for examples, adjacent a tumor, normal tissue or any other area of interest. The device may be identified by imaging techniques that measure, for examples, radio-opacity, ultrasound, magnetic or other characteristics that may be imaged. The imagable properties of the device maybe integral in its construction or may be added to the device in order to make it imagable. In one embodiment, the device may be situated in the body as part of an array or constellation of imagable markers. One or more of the imagable markers may also be a sensor device.

The device may include one or more sensor elements that sense one or more of a variety of physiological parameters, for examples, radiation dose, temperature, pH, metabolism, oxygenation. In one embodiment, the device may record and/or transmit such measurements, for example, by telemetric technology. Similarly, the device may respond to external signals (e.g., electrical, optical, ultrasonic, magnetic) or be programmed to respond to internally received signals that are being measured. The location of each of the sensor elements within the sensor device may be determined relative to one or more markers discussed in further detail below.

In one embodiment, the device may be configured to respond to a signal, for example, by release of a therapeutic drug enclosed within the device. For example, the device may respond to an external signal to become "activated" to produce a secondary local signal that causes release of therapeutic or diagnostic drugs that are encapsulated in other small containers injected or otherwise implanted into the body.

The sensor device(s) may be localized using image processing software. In one embodiment, this process may involve analysis of images taken from different perspectives. The location in the body of the imaged device(s) may be related to an array of markers that can, in turn, be related to various anatomical locations viewed by an imaging method. Accordingly, the location of the sensor device can be known relative to anatomical landmarks. Movement of the sensor device caused by motion of the part of the body in which it is located can also be measured so that location of the sensor device over an integral period of time can be directly known or can be mathematically modeled and predicted.

In one embodiment, orientation of the sensor device can also be determined through the use of multiple markers or multiple imaging properties. For example, markers may be placed on various locations on the device or in different patterns on the device. Different sections of the casing of the sensor device may be fabricated to have different imaging properties. If several sensor devices are placed in the body, they may each have different imaging markers or imaging properties, thereby making it possible to determine specific device location as well as a device's orientation.

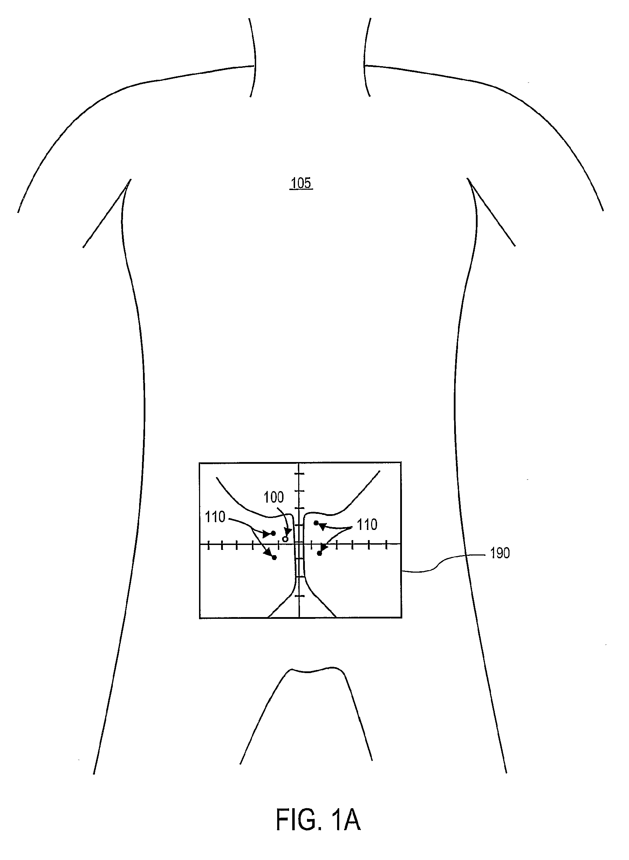

Figure 1A illustrates an enlarged imaged prostrate area of a patient's body having a sensor device and a plurality of markers (e.g., marker seeds). The sensor device 100 and the marker 110 are situated in or near an area of interest in body 105. In one embodiment, sensor device 100 may be situated within a volume defined by the array of markers seeds 110 as illustrated in Figure 1A. In an alternative embodiment, sensor device 100 may be situated outside a volume define by the array of markers 110.

For example, the area of interest may be a target volume in body 105 containing a prostate with a tumor cell population as illustrated in Figure 1A. An array of markers 110 may be implanted near the prostrate with the sensor device 100 situated within a volume defined by the array of markers 110. The sensor device 100 may be situated in the prostate to measure the dose of treatment radiation received. Although, conventional imaging techniques can locate a sensor device, it may be desirable to know the sensor device's precise position in the body 105 and, in particular, relative to other anatomical landmarks. For example, if the sensor device 100 is implanted in the prostate to monitor radiation dose delivered to a prostrate tumor, then the device's proximity to other anatomical landmarks (e.g., the rectal wall) would be desirable to know in order to extrapolate or otherwise determine radiation delivered to these other anatomical landmarks and minimize damage to such areas from subsequent the radiation treatment.

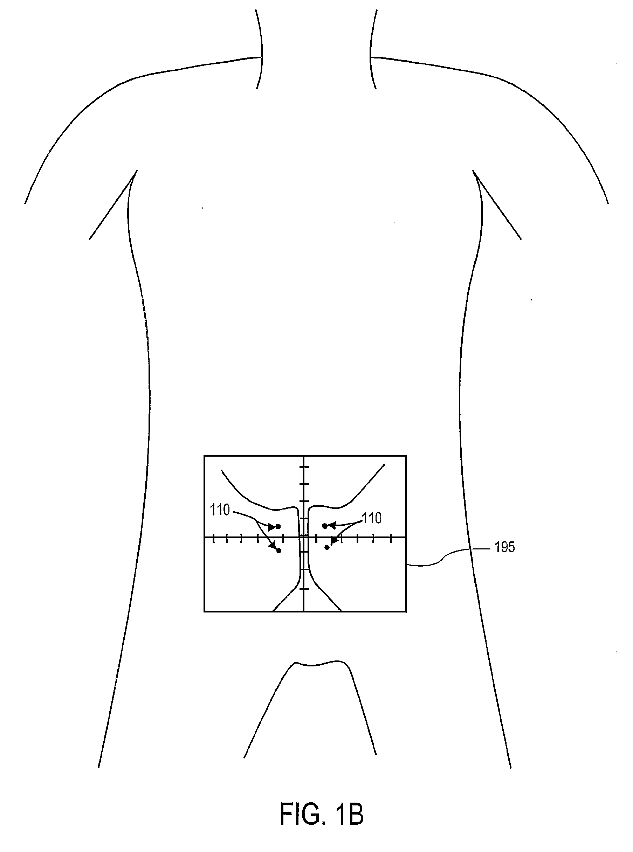

It should be noted that Figures 1A and 1B illustrate a prostrate area only for ease of discussion and that the invention is not limited to use only in a prostate area. In alternative embodiments, the area of interest may include any other area in the body such as other organs (e.g., liver or lung), a tumor, normal tissue, etc.

Sensor device 100 may sense one or more of a variety of physiological parameters, for examples, radiation dose, temperature, pH, metabolism, oxygenation. Continuing the example above, sensor device 100 may be used to monitor radiation dose delivered to tumor cells of the prostate. In one embodiment, the sensor device 100 may record and/or transmit such measurements, for example, by telemetric technology. Sensor and telemetric technology is known in the art; accordingly, a detailed discussion is not provided.

The sensor device 100 may respond to external signals (e.g., electrical, optical, ultrasonic, magnetic) or be programmed to respond to internally received signals that are being measured. In one embodiment, sensor device 100 may be configured to respond to a signal, for example, to release a therapeutic drug (e.g., chemo therapy for the prostate tumor) enclosed with the sensor device 100. In another embodiment, for another example, sensor device 100 may respond to an external signal to become "activated" to produce a secondary local signal that causes release of therapeutic or diagnostic drugs that are encapsulated in other devices (not shown) that have been injected or otherwise implanted into the body 105.

The markers 110 are intended to remain in position relative to the target tissue volume so that an imaging system can detect the markers as discussed below. In one embodiment, for example, the sensor device and/or the markers 110 may be placed in the needle of a biopsy syringe. The needle is injected into a patient's body and the sensor device and/or marker seed 110 is expelled from the needle into body tissue. Alternatively, other methods may be used to implant the sensor device and/or the markers 110, such as surgically.

During treatment, for example, a short x-ray exposure may be used to form an image for the purpose of imaging. In such an image, only bone and airways are readily discernable and soft-tissue delineation is limited. However, markers 110 placed within the target volume, such as the prostate area illustrated in Figure 1A, act as a facsimile for the target. The sensor device 100 and/or markers 110 may be imaged using one of several modalities, for examples, kilo voltage x-rays or mega voltage x-rays, ultrasound, or MRI. In one embodiment, the markers 110 may be used to determine an internal coordinate system and the location of the sensor device 100 may be determined relative to such an internal coordinate system.

In one embodiment, markers 110 may be marker seeds. Marker seeds may be cylindrical in shape with a length in the approximate range of 3.0 and 6.0 millimeters and a diameter in the approximate range of 0.5 and 3.0 millimeters. In alternative embodiments, the marker seeds may have other shapes (e.g., rectangular, spherical, etc.) and other dimensions. It should be noted that markers 110 are not limited to only markers seeds. Alternatively, other types of marker devices having imagable properties may be utilized as markers 110, for examples, surgical clips and orthopedic screws.

Conventional marker seeds have been made from various materials, for examples, gold and platinum due to their high density, high atomic number and biological compatibility. Because marker seeds typically are completely inactive, they tend not to do any injury to the body or cause discomfort to the patient. It may be desirable that markers 110 do not move relative to the target volume once implanted in the patient. In one embodiment, one or more of the markers 110 may be completely solid with a smooth surface or porous throughout its entire volume. Alternatively, markers 110 having a combination of dense material and porous material may be used to promote imaging detectability along with tissue adhesion.

Alternatively, other materials (e.g., tungsten or tantalum) and combinations of materials may be used for the markers 110. For example, if MRI imaging is to be used, the material(s) for the markers 110 may be chosen to be particularly effective in MRI applications. The markers 110 may be generated from materials chosen to minimize perturbation of a magnetic field. In one such embodiment, the markers 110 may be made from a combination of materials having magnetic susceptibilities of opposite sign. When a diamagnetic material (e.g., gold) is placed in an external magnetic field, it tends to exclude the magnetic field from the interior of the metal. Magnetic field lines are deviated so that a greater number of field lines pass around rather than through the metal when compared to the unperturbed magnetic field pattern. Conversely, paramagnetic materials (e.g., platinum and tantalum) in an external magnetic field will perturb the magnetic field in the opposite direction to diamagnetic material, so that the magnetic field lines are deviated so as to increase the number of field lines passing through the paramagnetic material.

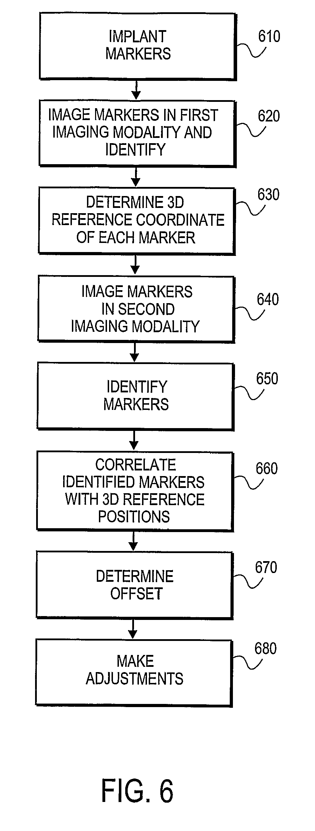

In one particular embodiment, the markers 110 are constructed of a material(s) such that they may be imaged using two or more modalities (by imaging techniques that measure, for examples, radio-opacity, sonic, magnetic or other material characteristics), as illustrated by Figures 1A and 1B. Figure 1B illustrates a sensor device not imagable in a second modality and an array of markers that are imagable in the second modality. In one embodiment, both the markers 110 and the sensor device 100 may be imaged using a first modality as illustrated by enlarged image 190 in Figure 1A. The image of the array of markers 110 may used to establish an internal coordinate system and the position of the sensor device 100 may be identified relative to one or more markers 110 in the established coordinate system, as discussed below in relation to Figure 6.

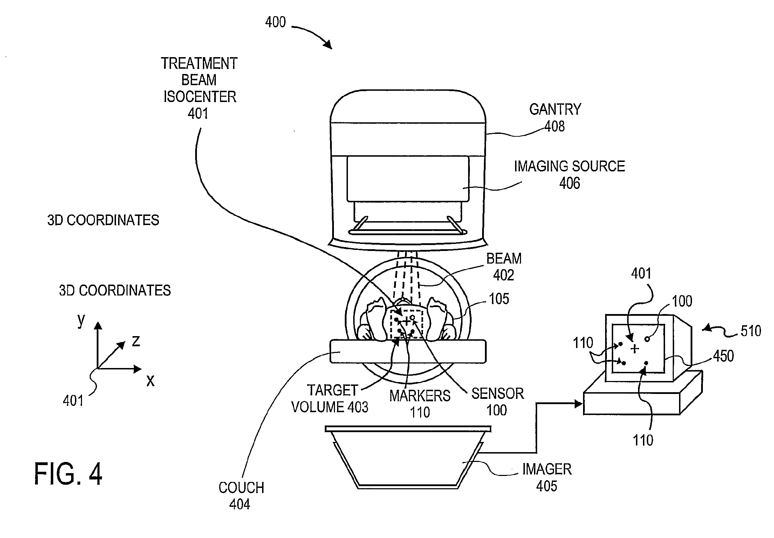

In the second modality, the markers 110 may also be imaged as illustrated by enlarged image 195 of Figure 1B, however, the sensor device 100 may not be imagable in this second modality as shown by the absence of sensor device 100 in enlarged image 195 of Figure 1B. In such an embodiment, the senor device 100 may be identified in the previously established coordinate system using image processing software to relate the positions of the array of markers seeds in the second imaging modality with their positions in the first imaging modality. The location in the body 105 of sensor device 100 imaged in the first modality is determined. When the position of the array of markers 110 in the second modality (illustrated by enlarged image 195) is identified in the coordinate system, the location of the sensor device 100 may be then calculated in the internal coordinate system (i.e., relative to one or more markers 110) and displayed with a computing system as discussed below in relation to Figure 4. The localization process is discussed in more detail below in relation to Figure 6.

As such, even though sensor device 100 cannot be imaged in second modality 195 of Figure 1B, the location of sensor device 100 in body 105 may be known relative to the array of markers 110. This, in turn, can be related to various anatomical landmarks viewable by the imaging modalities. Accordingly, the location of sensor device 100 can also be known relative to anatomical landmarks. Movement of the senor device 100 caused by, for example, motion of the part of the body in which sensor device 100 is situated can also be measured so that location of the device over an integral of time can be directly calculated or mathematically modeled and predicted. Tracking 3D position verses time may be performed as discussed below in relation to Figure 6. In one embodiment, the resulting trajectory of the markers may then be processed using a predictive filter, for example, as discussed in pending U.S. patent application 09/178,383 titled, "METHOD AND SYSTEM FOR PREDICTIVE PHYSIOLOGICAL GATING OF RADIATION THERAPY," Alternatively, other predictive filters known in the art may be used.

The position of internal body areas of interest constantly change due to, for examples, deformation of elastic structures (e. g. , organs) caused by normal fluctuations in respiration and muscle motion or by progression of disease (e. g., intra-cranial swelling). Such prevents areas (e. g. , organs) from remaining in a fixed position and makes it more difficult to aim treatment radiation at a precise point (e. g. , tumor). If the sensor device 100 is situated in such anatomic areas of body 105 that distort, then sensor device 100 may not be located in the same fixed position relative to an external reference source. If the array of markers seeds 110 is also located in the anatomic area that distorts, then by relating the position of the sensor device 100 to the array of markers seeds 110, a more accurate position of the sensor device 100 within the body 105 may be determined. More accurately knowing the location of the sensor device 100 in body 105 may facilitate measurement and/or delivery of, for example, radiation in certain areas in order to ensure that a target volume (e. g. , tumor) receives sufficient radiation and that injury to the surrounding and adjacent non-target volumes (e. g. , healthy tissue) is minimized.

In another embodiment, the array of markers 110 may be used either with or without sensor device 100 to determine the position of an anatomical landmark using a system that can directly image the array of markers 110 but, perhaps, not the anatomical landmark. In such an embodiment, an anatomical landmark (e. g. , bone, organ, or other body structure) is imaged with a first imaging modality and its location in body 105 related to the array of markers 110 that are also imagable with the first imaging modality. The imaging system generates an internal coordinate system based on the array of markers seeds 110 and determines the location of the anatomical landmark in the coordinate system. For example, if an ultrasound imaging system is used, then the imaging system can detect the position of the anatomical landmark and the positions of markers 110 using ultrasound techniques. An internal coordinate system may be calculated using the detected markers. Based on the position of the markers 110, the exact position of the anatomical landmark can be calculated relative to the internal coordinate system (e.g., relative to at least one of the markers).

At a following session, the array of markers 110 may be imagable in a second imaging modality 195 but not the anatomical landmark. However, even though the anatomical landmark cannot be imaged in second modality, the location of anatomical landmark may still be determined in the coordinate system by its previously determined positional relation to the markers 110. As such, because the markers 110 are imagable in second modality 195 of Figure 1B, the position of the anatomical landmark can be determined based on the established internal coordinate system.

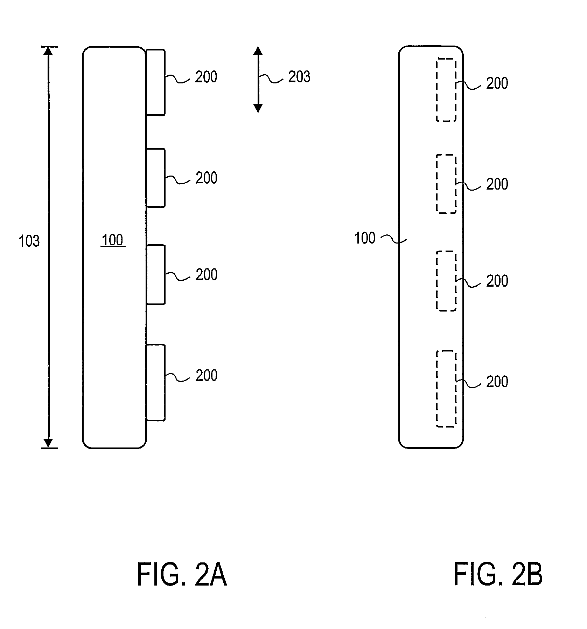

Figure 2A illustrates an embodiment of a sensor device having one or more markers disposed on its casing. In this embodiment, sensor device 100 includes multiple markers 200 that are coupled to the casing of the sensor device. Sensor device is shown with four markers only for ease of illustration. In alternative embodiments, sensor device 100 may have more or less than four markers 200 or no markers at all. Although Figure 2A illustrates markers 200 disposed along length 103 of sensor device 100, the markers 200 may be disposed in any configuration on sensor device 100. In one embodiment, length 103 may be, for example, less than 26 millimeters. Alternatively, sensor device 100 may have another length. In another embodiment, markers 200 may be disposed in sensor device 200 as illustrated in Figure 2B.

In one particular embodiment, for example, the marker seeds 200 may be cylindrical in shape and have a length 203 in the approximate range of 3.0 and 6.0 millimeters and a diameter in the approximate range of 0.5 and 3.0 millimeters. In alternative embodiments, marker seeds 200 may have other shapes (e.g., rectangular, spherical, etc.) and other dimensions.

It is desirable that the sensor device 100 does not move relative to the target volume once implanted in the patient. In one embodiment, one or more of the markers 200 may be completely solid with a smooth surface or porous throughout its entire volume. Alternatively, one or more of the markers 200 may have a combination of dense material and porous material that may be used to promote imaging detectability along with tissue adhesion.

Alternatively, other materials (e.g., tungsten or tantalum) and combinations of materials may be used for the markers 200. For example, if MRI imaging is to be used, the material(s) for the markers 200 may be chosen to be particularly effective in MRI applications. The markers 200 may be generated from materials chosen to minimize perturbation of a magnetic field. In one such embodiment, the marker may be made from a combination of materials having magnetic susceptibilities of opposite sign as discussed above with respect to markers 110 of Figure 1A.

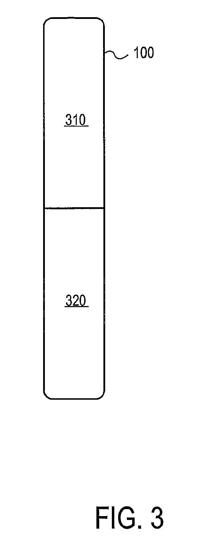

Figure 3 illustrates one embodiment of a sensor device having a casing with multiple imaging properties. In one embodiment, for example, different sections (e.g., 310 and 320) of the casing of sensor device 100 may be fabricated to have different imaging properties.

As previously noted, the markers 200 and/or imaging properties may be disposed in various locations on sensor device 100 and in different patterns on sensor device 100. As such, the orientation of sensor device 100 can be determined through the use of multiple markers 200 of Figures 2A, 2B or multiple imaging property regions (e.g., 310 and 320) of Figure 3. If several senor devices 100 are placed in the body 105, they may each have different marker properties such as through means of multiple imaging markers disposed thereon/therein or multiple imaging properties integral in the sensor device's construction (e.g., part of its casing), thereby making it possible to determine specific device location as well as a device's orientation.

One or more of senor device 100 and markers 110 may be localized by an image system as illustrated in Figure 4. Figure 4 illustrates one embodiment of a system 400 that represents a treatment planning and/or delivery system. While at times discussed in relation to a treatment planning system, system 400 also represents a treatment delivery system. As such, beam 402 may represent both an imaging beam and a treatment beam depending on the context of the discussion. The planning system and the treatment system may be physically different machines or incorporated together within a machine. In one embodiment, for example, the delivery system may be, for examples, a Clinac® Linear Accelerator and a Multi-Leaf Collimator (MLC™) available from Varian Medical Systems, Inc. of California. The configuration of system 400 shown is only for ease of discussion and illustration purposes and various other configuration known in the art may be used, for example, imager 405 may be located on a gantry rather than incorporated into treatment table 404. It should also be noted that the imaging system 400 may be discussed in relation to particular imaging modalities only for ease of discussion and that other imaging modalities may be used as mentioned above.

Shown in Figure 4 is a body 105 supported by a treatment table 404 and an imager 405. An imaging source (e.g., kilo voltage x-rays, mega voltage x-rays, ultrasound, MRI, etc.) 406 may be located, for example, in gantry 408 and imager 405 may be located, for example, beneath body 105 opposite that of the imaging source 406. The imager 405 is positioned to detect and receive the beam 402 generated by imaging source 406. The output images of the imager 405 are sent to computer 510.

Computer 510 receives the output images of imager 406 that includes the image of at least one of markers 110, sensor device 100 and/or an anatomical landmark. The images received from imager 406 are used by computer 510 to develop a coordinate system for markers 110. At a first treatment session using a first imaging modality, markers seeds 110 and a sensor device 100 (and/or an anatomical landmark) are detected and the coordinates for each of the markers 110 are determined and stored in computer 510. Thereafter, at a subsequent session, using a different imaging modality, system 400 can detect the markers 110 and determine their position in the coordinate system by comparison to stored data in computer 510. The position of the sensor device 110 and/or anatomical landmark not imagable in the second modality may then be determined by computer system 510 through using the previously established coordinate system, as discussed above.

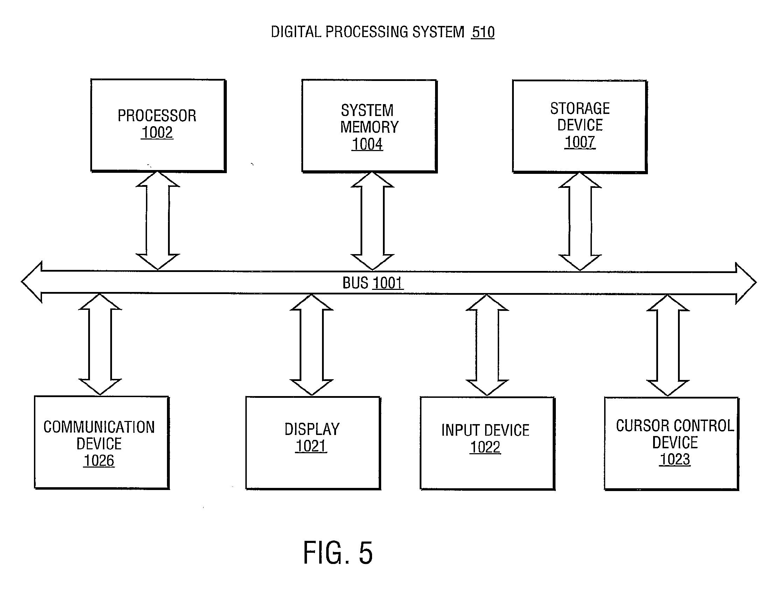

Figure 5 illustrates one embodiment of digital processing system 510 of Figure 4 representing an exemplary workstation, personal computer, laptop computer, handheld computer, personal digital assistant (PDA), closed-circuit monitoring box, etc., in which features of the present invention may be implemented.

Digital processing system 510 includes a bus or other means 1001 for transferring data among components of digital processing system 510. Digital processing system 510 also includes processing means such as processor 1002 coupled with bus 1001 for processing information. Processor 1002 may represent one or more general-purpose processors (e.g., a Motorola PowerPC processor and an Intel Pentium processor) or special purpose processor such as a digital signal processor (DSP) (e.g., a Texas Instruments DSP). Processor 1002 may be configured to execute the instructions for performing the operations and steps discussed herein. For example, processor 1002 may be configured to execute instructions to cause the processor to track vascular intervention sites.

Digital processing system 510 further includes system memory 1004 that may include a random access memory (RAM), or other dynamic storage device, coupled to bus 1001 for storing information and instructions to be executed by processor 1002. System memory 1004 also may be used for storing temporary variables or other intermediate information during execution of instructions by processor 1002. System memory 1004 may also include a read only memory (ROM) and/or other static storage device coupled to bus 1001 for storing static information and instructions for processor 1002.

A storage device 1007 represents one or more storage devices (e.g., a magnetic disk drive or optical disk drive) coupled to bus 1001 for storing information and instructions. Storage device 1007 may be used for storing instructions for performing the steps discussed herein.

In one embodiment, digital processing system 510 may also be coupled via bus 1001 to a display device 1021, such as a cathode ray tube (CRT) or liquid crystal display (LCD), for displaying information to the user. Such information may include, for example, graphical and/or textual depictions such as coordinate systems, markers, sensor devices and/or anatomical landmarks as illustrated by images 450 of Figure 4. An input device 1022, such as a light pen, may be coupled to bus 1001 for communicating information and/or command selections to processor 1002. Another type of user input device is cursor control 1023, such as a mouse, a trackball, or cursor direction keys for communicating direction information and command selections to processor 1002 and for controlling cursor movement on display 1021.

A communications device 1026 (e.g., a modem or a network interface card) may also be coupled to bus 1001. For example, the communications device 1026 may be an Ethernet card, token ring card, or other types of interfaces for providing a communication link to a network, such as a remote diagnostic or monitoring system, for which digital processing system 510 is establishing a connection.

It will be appreciated that the digital processing system 510 represents only one example of a system, which may have many different configurations and architectures, and which may be employed with the present invention. For example, some systems often have multiple buses, such as a peripheral bus, a dedicated cache bus, etc.

Figure 6 illustrates one embodiment of a method of localizing a landmark. In a treatment planning stage, markers 110 (and one or more of sensor device 100, if desired) are implanted in a target volume 403 of Figure 4, step 610. The implantation may be performed in a manner discussed above with respect to Figures 1A and 1B. Typically, as part of a tumor radiation treatment planning process, the isocenter of a treatment beam (having known size and shape), that will be used to treat a patient, is determined (e.g., by a physician) in order to accurately position the body, and hence a tumor, within a radiation beam during treatment. For example, during treatment planning a series of CT slices of the body 105 through the target volume 403 maybe taken. A physician may view a tumor in the CT slices (e.g., presented in either 2D or 3D) and define a boundary for the treatment volume 403 on computer 501 display of one or more of the CT slices. Treatment planning software known in the art may be used to calculate the isocenter 401 based on the defined boundary. In one embodiment, for example, Eclipse™ treatment planning software available from Varian Medical Systems, Inc. of California may be used. Alternatively, other treatment planning software may be used. Such accurate positioning maximizes the treatment radiation dose delivered to a tumor while minimizing the radiation dose to surrounding normal tissue.

In one embodiment, as part of a treatment planning process, the 3D coordinates of the implanted markers 110 may be localized relative to the isocenter 401 (hence the target volume 403) of the treatment machine beam 402. The markers 110 may be imaged in a first imaging modality, step 620, using, for example, a series of CT slices of the body105 through the target volume 403. CT measures the average x-ray absorption per volume element (voxel) in slices projected through body 105. A planning CT set where automated or user-assisted techniques known in the art may be used to identify the markers 110 in the CT slices (e.g., using computer system 510). Alternatively, the localization of the markers 110 may be performed in other imaging modalities and also at other times (e.g., before or after the treatment planning session). The first modality images may be imported to a computer system (e.g., computer system 510) for determination of the 3D reference coordinates of the markers 110.

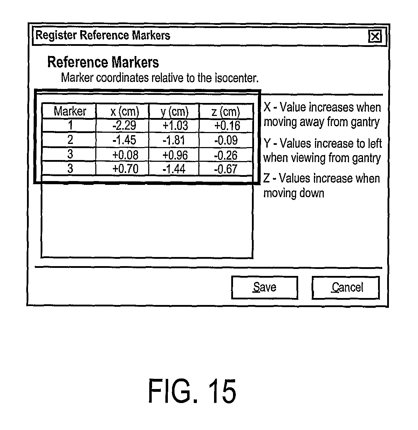

Using the information from step 620, and assuming the treatment beam isocenter 401 is known, the 3D reference coordinates of each marker 110 relative to the isocenter 401 may be determined using software techniques known in the art, step 630. In the embodiment where localization of the markers is performed prior to treatment planning (hence the isocenter 401 is not yet known), the voxel coordinates of the markers may be stored (e.g., in computer system 510) and translated to the reference coordinates relative to the isocenter 401 once the isocenter 401 is determined. The coordinates of the reference markers may be displayed to a user, for example, using a graphical user interface as illustrated, for one embodiment, in Figure 15. In this embodiment, for example, the 3D reference coordinates (x, y, z) of each marker relative to isocenter 401 are determined with: a positive x value increasing when the marker is farther away from gantry 408, a positive y value increasing when a marker is farther to the left when viewed from gantry 408; a positive z value increasing when a marker is farther down from gantry 408. Alternately, other positional relationships may be used for the coordinates.

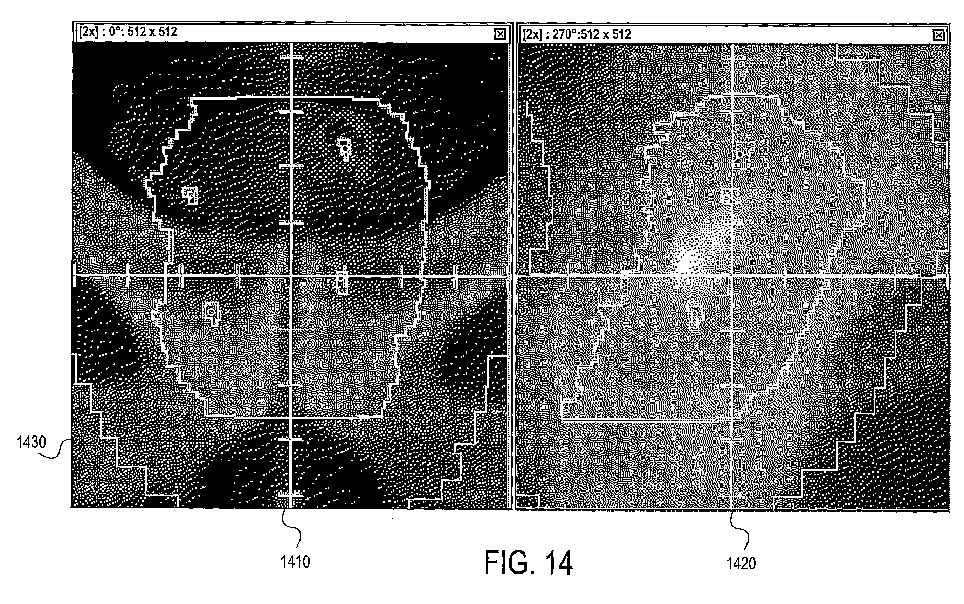

It should be noted that other methods may be used to localize the markers 110. In another embodiment, for example, digitally reconstructed radiographs (DRR) produced from different view angles using a CT set, as illustrated in Figure 14, can be used to localize the markers by triangulation methods known in the art. Figure 14 illustrates a first view angle 1410 and a second view angle 1420 being, for example, 270 degrees offset with respect to view angle A. The DRRs of Figure 14 may include the field shape (e.g., field shape 1430) of the markers. In yet another embodiment, the marker 110 locations may be manually entered by a user of the system using techniques such coordinate entry or marking through a graphical user interface.

As previously mentioned, daily treatment machine setup variation and various types of organ movement from that encountered in the treatment planning session contribute to uncertainty in the position of the target volume 403 relative to the treatment machine beam 402 isocenter 401 during a particular treatment session. In order to minimize any such positional offset, markers 110 are used to more closely align target volume 403 with the treatment beam 402. Since the 3D reference coordinates of each marker 110 relative to the planning isocenter 401 was determined in step 630 then, if the markers 110 are imagable during the treatment session, any offset of the markers 110 position with respect to the known beam isocenter at the time of treatment may be determined and corrected.

To achieve this, in a particular treatment session, the markers 110 are imaged in a second modality, step 640. The second modality may be the same as the first modality. Alternatively, the second imaging modality used to acquire the images in step 640 may be different than the first imaging modality. In one embodiment, the second modality images may be X-ray images acquired using, for example, a MV portal imager and/or a KV imager. It is assumed that the reference coordinates of the imager 405 are calibrated relative to the treatment machine isocenter 401.

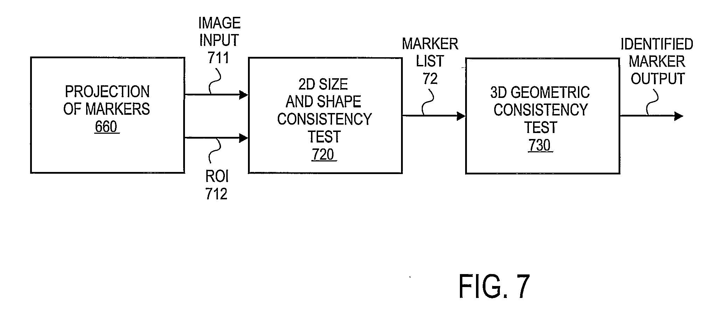

In step 650, the markers 110 (e.g., radio-opaque) in the second modality images (e.g., X-ray) are identified. It should be noted that the second modality images may contain non-marker objects or images that may be considered to be markers (false markers). In one embodiment, falsely detected markers may be removed from the set of identified markers, as discussed in relation to Figure 7.

In step 660, each marker110 identified in the second modality image of step 650 is correlated with its 3D reference position as determined in step 620 after projecting the marker from 3D to the 2D image domain based on the known geometry of the acquired image. In one embodiment, the identified markers 110 in step 650 are those that pass the consistency tests discussed below in relation to Figure 7. Alternatively, consistency tests need not be employed or other types of screening may be performed to arrive at a set of identified markers.

The 2D coordinates of the identified markers 110 are used to find the position and orientation of the marker set relative to the treatment machine isocenter 401, step 660. In one embodiment, the position and orientation of the markers 110 relative to the treatment machine isocenter 401 may be determined by triangulation from two or more images. For example, stereoscopic representations of a treatment volume 403 can be obtained by merging data from one or more imagers taken at different locations. Treatment couch 404 can position the patient and, thereby, a treatment volume 403, within a radius of operation for the treatment machine 400. At a single gantry 408 position, or through gantry rotation, multiple single images can be generated at different radial locations and any two images may be selected and merged by computer 510 into a stereoscopic representation of the treatment volume. The stereoscopic representation can be generated to provide 2D cross-sectional data for a selected radial position. The stereoscopic representation can be used to determine the 3D coordinates of the markers 110 relative to known treatment beam isocenter 401. Alternatively, other triangulation techniques may be used. Triangulation techniques are known in the art; accordingly a detailed discussion is not provided.

In an alternative embodiment, for another example, the position and orientation of the markers 110 relative to the treatment machine isocenter 401 may be determined using a single view position and orientation estimation of a rigid structure defined by the step 630 reference marker coordinates, as discussed in pending US patent application 10/234,658. The former embodiment method may be better suited for less rigid targets such as a prostate or liver. The later embodiment method may be effective for strictly rigid targets such as bony tissue. Alternatively, yet other methods may be used to determine the position and orientation of the marker set.

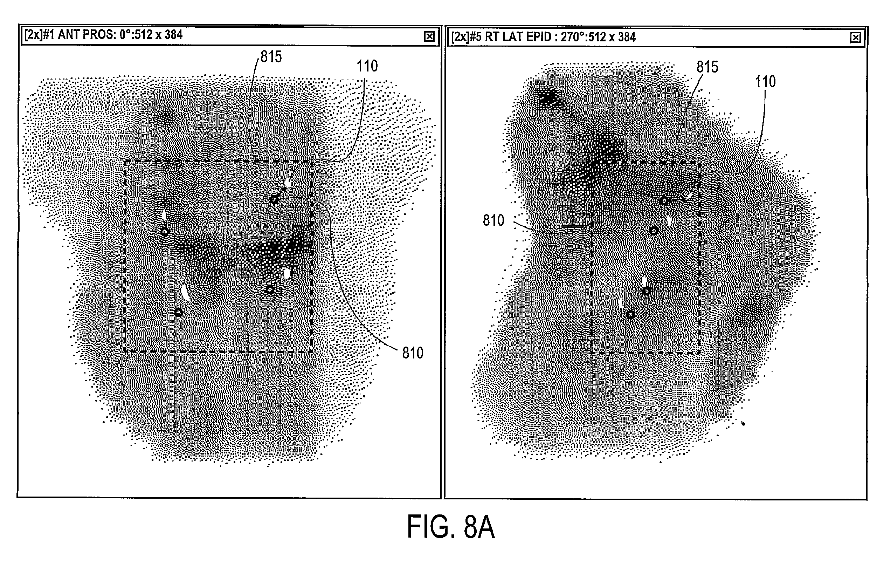

For the detected markers, the 3D coordinate of each marker 110 with its corresponding 3D reference coordinate (e. g. , 3D reference coordinate position 810 of Figure 8A) is compared to determine the offset between the two data sets (e.g., in the form of delta x, y, and z values), step 670. Ideally, if the patient body 105 were positioned perfectly, there should be no offset between the two data sets, i.e., the markers 110. In practice, however, there maybe some offset (e.g., offset 815) between the two sets as illustrated in Figure 8A.

It should also be noted that not all of the implanted markers 110 may be imaged or identified in step 650. The position of the unidentified markers in step 650 may be determined based on the positional relationship between the reference markers positions acquired in step 620. In one embodiment, a rigid body transform may be estimated that, when applied to the reference marker set, minimizes the means square error between the 3D coordinates of the identified markers 110. When the rigid body transform is applied to the reference marker set, including the markers that were not detected in the second imaging modality of step 650, an estimated position of the undetected markers in the second modality may be obtained. In one embodiment, for example, the undetected marker may actually be sensor device 100 (with or without marker properties) not imagable in the second modality 195 of Figure 1B or step 640 of Figure 6. In an alternative embodiment, for example, the undetected marker may actually be an anatomical landmark rather than one of the markers.

In step 680, based on the offset position and orientation differences between the reference marker set and treatment session's marker set, the needed adjustments to the patient setup (e.g., position and/or orientation of couch 404) and/or adjustments to the treatment beam 402 (e.g., gantry 408 angle, collimator rotation angle, etc.) may be estimated in order to achieve the best match between treatment geometry and the planned geometry for the target volume 403. It should be noted that offset information may be determined in other manners. In an alternative embodiment, for example, the center of mass (centroid) of both the reference marker set and treatment session's detected marker set may be calculated and compared to determine the positional offset between the two.

Figure 7 illustrates one embodiment of detecting a marker and removing a false marker in an image. In this embodiment, falsely detected markers in step 650 may be removed from the set of identified markers. As discussed above in relation to step 660, after projecting the markers 110 based on the known geometry of an acquired image, the image 711 and a region of interest (ROI) 712 for the image are provided to a 2D size and shape consistency test, step 720. The 2D size and shape consistency test is performed to identify markers in an image. In one embodiment, the 2D size and shape consistency test may be performed using an automatic detection algorithm utilizing a median filter and/or connected component analysis.

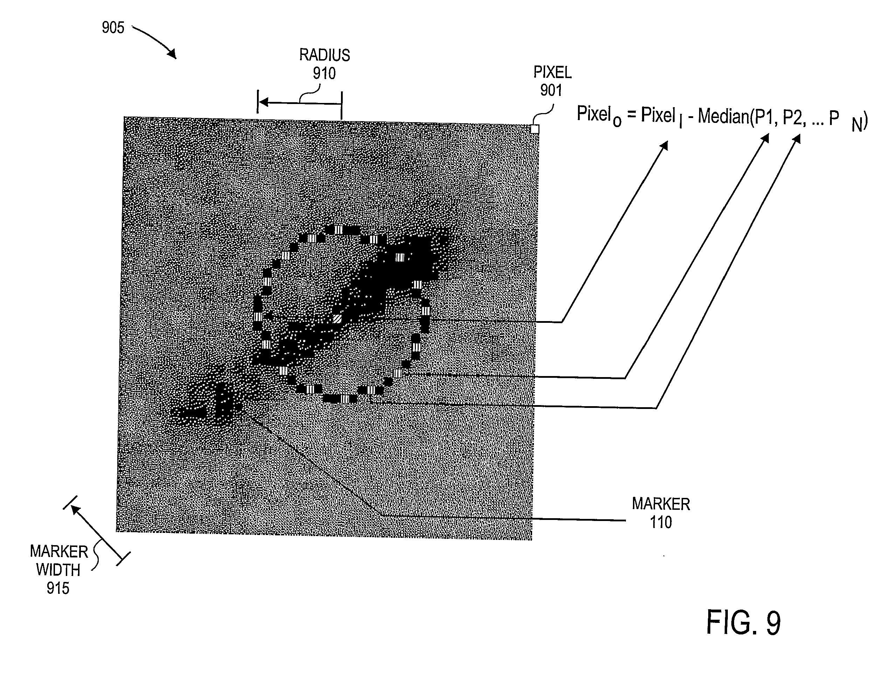

Figure 9 illustrates one embodiment of a median filtering of an image containing a marker. Median filter 905 may be used to filter intensity values of pixels of an image to determine whether a particular image pixel contains a portion of a marker (or other imagable object) or background noise.

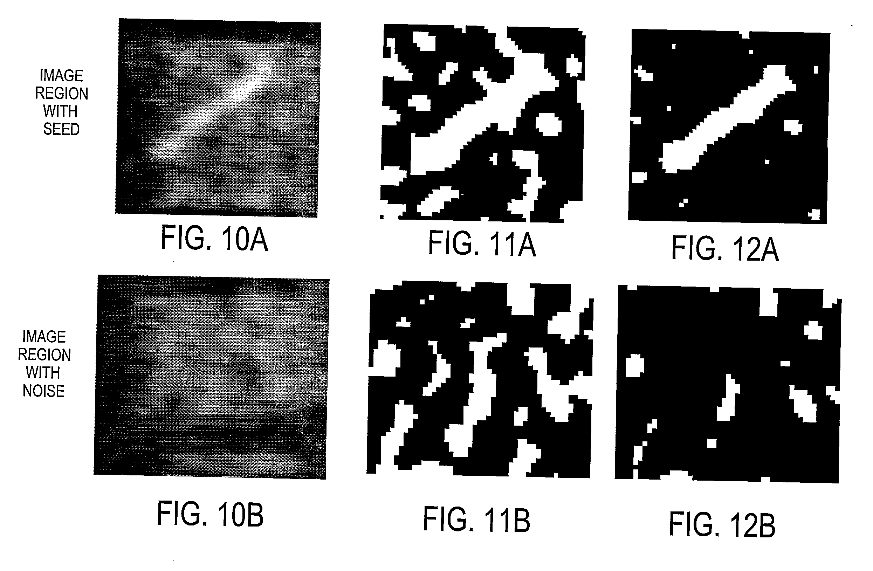

In this embodiment, for a certain number of pixels in an image (e.g., pixel 901, pixel_{I}, etc.), the median filter evaluates a certain number of perimeter pixels (e.g., P1, P2, PN, etc.) of an approximate circle, or "ring," (having a certain approximate radius) around that center pixel (e.g., pixel_{I}). The median filter 905 takes the median intensity values of the perimeter pixels (e.g., pixels P1, P2, etc.) and subtracts the median values from the evaluate center pixel (e.g., pixel_{I}) to output a filtered pixel intensity value Pixel_{O}. The Pixel_{O} values are used to generate a filtered image as illustrated in Figures 10-12 below. The effect of median filter 905 is to remove the background intensity noise of an image to produce a filter imaged with better visual distinction between markers 110 and the original image background, as illustrated in Figures 10A and 10B below.

In one particular embodiment, for example, N=16 (i.e., the ring median filter evaluates 16 perimeter pixels). The radius 910 is selected to be greater than half the marker width 915. In one particular embodiment, for example, the radius 910 of the circle is selected to be approximately 10 pixels based on known size of a pixel and the known size of an implanted marker 110. In another embodiment, the ring diameter (2 x radius 910) is selected to be approximately 2.6 times the marker width 915 in pixels, which is independent of pixel 901 size. This causes the median statistics to represent the median of the background (non-marker) pixels even when the ring intersects with marker 110.

Alternatively, other evaluation region perimeter shapes (e.g., elliptical, rectangular, square, etc.), dimensions, number of pixels evaluated in an image, number of perimeter pixels, etc. may be used. In an alternative embodiment, the other filtering (e.g., mean filtering) and background subtraction techniques known in the art may be used.

Figures 10A and 10B illustrate image ROIs containing a marker and no marker (just background), respectively, after the use of a median filter 905 in the 2D size and shape consistency test 720. As discussed above in relation to Figure 7, in one embodiment, the 2D size and shape consistency test 720 may also utilize a connected component analysis to further screen markers 110 from the background of an image. In a connected component analysis, all connected components in an image are found. In order to remove noise contours, regions with small areas are filtered according to a threshold. The threshold may be decreased or increased based on the size of the markers 110. Figures 11A and 11B illustrate image ROIs containing a marker and no marker (just background), respectively, after a connected component analysis with a low threshold. Figures 12A and 12B illustrate image regions containing a marker and no marker (just background), respectively, after a connected component analysis with a higher threshold than used for the images of Figures 11A and 11B. Connected component analysis techniques are known in the art; accordingly a detailed discussion is not provided.

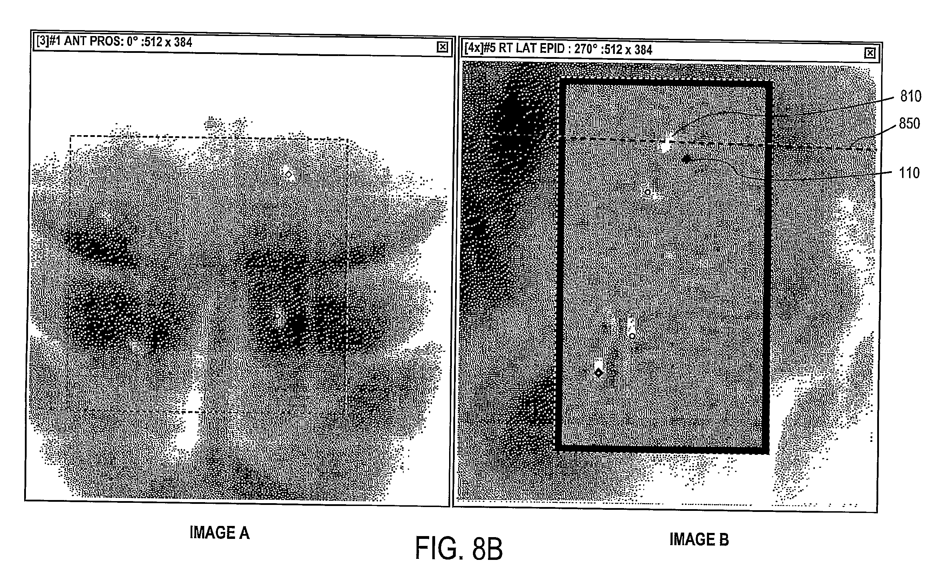

Referring back to Figure 7, after 2D size and shape consistency test 720 is performed, a marker list 721 of the identified markers is then output to a 3D geometric consistency test 730. The 3D geometric consistency test 730 may be used to screen out false markers. In one embodiment, the 3D geometric consistency test 730 may be performed using an epipolar coincidence constraint. This condition is based on the availability of a pair of stereo images, as illustrated in Figure 8B. A point (pixel position) in image A is back projected as a line in 3D space. The image of this 3D line in the other image B of the stereo pair is the epipolar line 850. Therefore, when a marker 110 is detected in one image (e.g., image A), its projection in the other image (e.g., image B) must lie on, or very close to, the epipolar line 850 of the first image position of the marker 110. The degree of expected closeness depends on the amount of calibration error. The epipolar constraint may be used to define search areas for detection in one image based on a detection in another image and to discard false detections that do not satisfy the constraint. At times a marker 110 may be detected with high confidence in one image while being barely visible in the other image of the stereo pair. In one embodiment, the epipolar line 850 may be used to setup a search region with lower detection threshold in the image where the marker 110 is less visible.

The 2D size and shape consistency test and the 3D geometric consistency test may be performed for either a region of interest, or one or more markers. It should be noted that a subset or variation of the above steps of Figures 6 and 7 may be used for cases with fewer images and/or fewer markers.

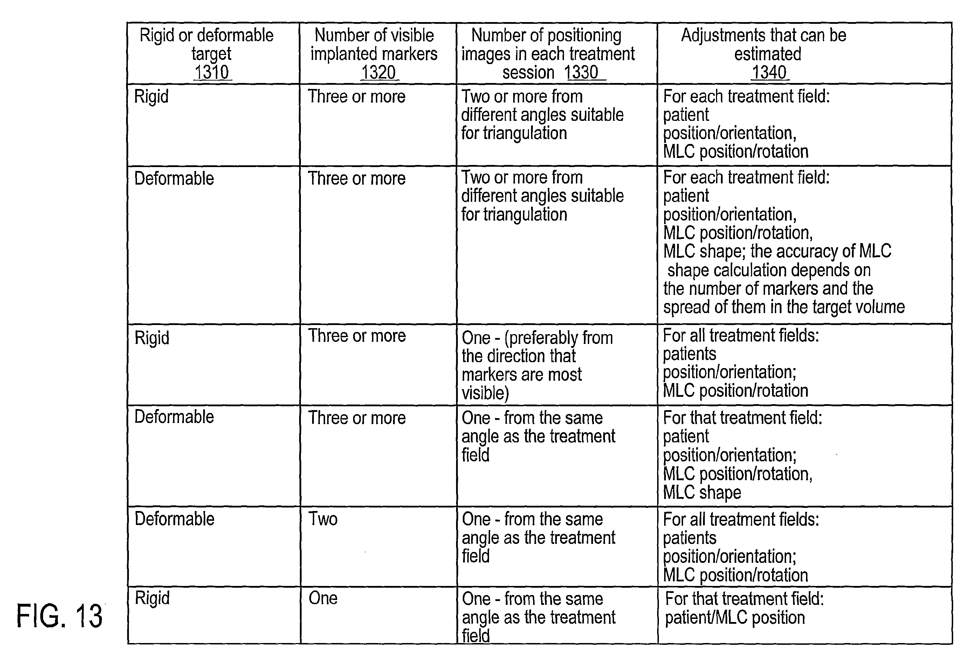

As previously mentioned, by comparing the position of the markers 110 in a treatment session with their reference position, adjustments to the patient body 105 position and orientation, and/or treatment beam 402 direction and shape may be calculated in such a way that the actual target volume 403 relative to the treatment beam 402 is as close as possible to the planned target volume 403 with possible adjustments to the shape of the beam 402 to accommodate possible landmark (e.g., tumor) deformations. The patient and beam adjustments that can be estimated, and the accuracy of the estimation, vary depending on the number of the implanted markers 110, the number of markers 110 that are visible in the image generated in the second imaging modality in the treatment session, the number of images acquired in a treatment session, and the rigidity of the target volume 403. Example cases include (but are not limited) to the ones discussed below in relation to the table of Figure 13.

Figure 13 is a table illustrating the relationship between various localization parameters. Table 1300 includes columns 1310, 1320, 1330, and 1340. Column 1310 contains information on the rigidity (e.g., how fixed is the spacing between markers 110 over the treatment course) of a target volume 403. Column 1320 contains the number of implanted markers 110 that may be visible in an image. Column 1330 contains adjustments to the patient body 105 and/or treatment beam 402 positioning that can be estimated. Column 1340 contains the number of positioning images in each treatment session that may be necessary.

In one embodiment, the adjustments (e.g., patient and/or beam) that can be estimated (and the accuracy of the estimation) and the number of positioning images that may be required in a treatment session may be based on (1) the rigidity of the target; and (2) the number of visible markers in an image.

The rigidity of a target is may be defined in relative terms. The effectiveness of implementing some of the estimated adjustments mentioned in the above table depends on how rigid the target is. For example the rigidity assumption may be generally accepted for markers 110 attached to a bony target. In contrast, a prostate may deform and change in size during the course of treatment to some greater extent than a bony target. To treat the prostate as a deformable target and actually adjust the shape of the MLC for each field of each treatment session, a larger number of markers 110 spread somewhat uniformly throughout the target volume 403 may be required. MLC are discussed, for example, in U. S. patents 5, 166, 531 and 4, 868, 843.

In the foregoing specification, the invention has been described with reference to specific exemplary embodiments thereof. It will, however, be evident that various modifications and changes may be made thereto without departing from the scope of the invention as set forth in the appended claims. The specification and drawings are, accordingly, to be regarded in an illustrative sense rather than a restrictive sense.

## Claims

1. A method, comprising:
generating a first image data set using a first imaging modality, the first image data set comprising a first plurality of images of a target volume within a human body, and of a plurality of markers (110, 200) internally implanted in proximity or relative to the target volume;
generating a treatment plan based on a selected treatment plan center and the first image data set;
determining a first internal three-dimensional coordinate system based on locations of the plurality of markers (110, 200) in the first image data set and the selected treatment plan center;
determining a first set of three-dimensional coordinates of locations of the plurality of markers in the first image data set based on the first internal coordinate system;
generating a second image data set using a second imaging modality, the second image data set comprising a second plurality of images of the target volume within the human body, and of the plurality of markers (110, 200) internally implanted in proximity of relative to the target volume;
determining a second internal three-dimensional coordinate system based on locations of the plurality of markers (110, 200) in the second image data set and a treatment machine isocenter of the second imaging modality;
determining a second set of three-dimensional coordinates of locations of the plurality of markers in the second image data set based on the second internal coordinate system;
determining an offset (815) between the first set of three-dimensional coordinates of the plurality of markers and the second set of three-dimensional coordinates of the plurality of markers; and
adjusting the treatment plan based on the determined offset (815).

2. The method of claim 1, wherein the first image data set comprises an anatomical structure in proximity of the target volume; and further comprising:
determining a position of the anatomical structure with respect to the target volume in the second image data set, based on the second image data set and the internal coordinate system.

3. The method of claim 1, wherein the second imaging modality is different from the first imaging modality.

4. The method of claim 1, wherein the second imaging modality is the same as the first imaging modality.

5. The method of claim 4, wherein the first imaging modality is an x-ray imaging modality, the second imaging modality is an x-ray imaging modality coupled to a treatment machine having the treatment beam source and the machine center of a high energy beam of radiation of the treatment machine beam source.

6. The method of claim 1, wherein the first imaging modality is CT and the second imaging modality is kilo volt (kV) imaging; and wherein the markers (110, 200) are x-ray imagable, internal, and inserted into a patient (105).

7. The method of claim 1, wherein the first imaging modality is a planning machine imager to show the target volume in the human body for later treatment and the planned selected treatment plan center where it is desired to align the treatment beam at the treatment machine; wherein the second imaging modality is a treatment machine imager to show the target region of the patient during treatment and to show the machine center where it is known to be at the treatment machine computer for the treatment beam at the treatment machine.

8. The method of claim 1, wherein the first imaging modality is a planning machine imager to show a target region in a patient (105) for later treatment and the selected treatment plan center where it is desired to align the treatment beam at the treatment machine; wherein the second imaging modality is coupled to a treatment machine to show the target region of the patient and to show the machine center of the second imaging modality where it is known to be at the treatment machine computer for the treatment beam at the treatment machine.

9. The method of claim 8, wherein the offset between the first coordinates and the second coordinates is determined by aligning at least the selected treatment plan center with the machine center.

10. The method of claim 8, wherein adjusting the treatment plan comprises:
determine a support adjustment to adjust a support supporting the human body (1) based on the determined change in position of the target relative to the machine center and (2) relative to the plurality of markers (110, 200); and
moving the support based on the support adjustment to minimize the determined offset.

11. The method of claim 10, wherein determining the offset includes calculating a relative position and a relative orientation of at least one of the plurality of markers (110, 200) imaged in the second modality, relative to the machine center, and
wherein determining the support adjustment includes using the relative orientation and the relative position of the markers relative to the machine center.

12. The method of claim 1, wherein determining the offset includes aligning the selected treatment plan center and the machine center to align a planned target with the treatment machine beam, even though the markers are not aligned;
wherein the offset identifies a rotational difference in the marker offset (815); and
wherein the offset identifies an x, y, z shift in the marker offset.

## Patentansprüche

1. Verfahren, das Folgendes beinhaltet:
Erzeugen eines ersten Bilddatensatzes mit einer ersten Bildgebungsmodalität, wobei der erste Bilddatensatz eine erste Mehrzahl von Bildern eines Zielvolumens in einem menschlichen Körper und einer Mehrzahl von Markern (110, 200) umfasst, die intern nahe oder relativ zu dem Zielvolumen implantiert sind;
Erzeugen eines Behandlungsplans auf der Basis eines gewählten Behandlungsplanzentrums und des ersten Bilddatensatzes;
Ermitteln eines ersten internen dreidimensionalen Koordinatensystems auf der Basis von Orten der Mehrzahl von Markern (110, 200) in dem ersten Bilddatensatz und dem gewählten Behandlungsplanzentrum;
Ermitteln eines ersten Satzes von dreidimensionalen Koordinaten von Orten der Mehrzahl von Markern in dem ersten Bilddatensatz auf der Basis des ersten internen Koordinatensystems;
Erzeugen eines zweiten Bilddatensatzes mit einer zweiten Bildgebungsmodalität, wobei der zweite Bilddatensatz eine zweite Mehrzahl von Bildern des Zielvolumens innerhalb des menschlichen Körpers und der Mehrzahl von Markern (110, 200) umfasst, die intern nahe oder relativ zu dem Zielvolumen implantiert sind;
Ermitteln eines zweiten internen dreidimensionalen Koordinatensystems auf der Basis von Orten der Mehrzahl von Markern (110, 200) in dem zweiten Bilddatensatz und einem Behandlungsmaschinen-Isozentrum der zweiten Bildgebungsmodalität;
Ermitteln eines zweiten Satzes von dreidimensionalen Koordinaten von Orten der Mehrzahl von Markern in dem zweiten Bilddatensatz auf der Basis des zweiten internen Koordinatensystems;
Ermitteln eines Versatzes (815) zwischen dem ersten Satz von dreidimensionalen Koordinaten der Mehrzahl von Markern und dem zweiten Satz von dreidimensionalen Koordinaten der Mehrzahl von Markern; und
Justieren des Behandlungsplans auf der Basis des ermittelten Versatzes (815).

2. Verfahren nach Anspruch 1, wobei der erste Bilddatensatz eine anatomische Struktur nahe dem Zielvolumen umfasst; und das ferner Folgendes beinhaltet:
Ermitteln einer Position der anatomischen Struktur mit Bezug auf das Zielvolumen in dem zweiten Bilddatensatz auf der Basis des zweiten Bilddatensatzes und des internen Koordinatensystems.

3. Verfahren nach Anspruch 1, wobei sich die zweite Bildgebungsmodalität von der ersten Bildgebungsmodalität unterscheidet.

4. Verfahren nach Anspruch 1, wobei die zweite Bildgebungsmodalität dieselbe ist wie die erste Bildgebungsmodalität.

5. Verfahren nach Anspruch 4, wobei die erste Bildgebungsmodalität eine Röntgenbildgebungsmodalität ist, die zweite Bildgebungsmodalität eine Röntgenbildgebungsmodalität gekoppelt mit einer Behandlungsmaschine mit der Behandlungsstrahlenquelle und dem Maschinenzentrum eines Hochenergiestrahls der Behandlungsmaschinenstrahlenquelle ist.

6. Verfahren nach Anspruch 1, wobei die erste Bildgebungsmodalität CT ist und die zweite Bildgebungsmodalität Kilovolt-(kV)-Bildgebung ist; und wobei die Marker (110, 200) durch Röntgenstrahlen abbildbar, intern und in den Patienten (105) eingeführt sind.

7. Verfahren nach Anspruch 1, wobei die erste Bildgebungsmodalität ein Planungsmaschinen-Imager ist, um das Zielvolumen im menschlichen Körper für eine spätere Behandlung und das geplante gewählte Behandlungsplanzentrum zu zeigen, wo der Behandlungsstrahl an der Behandlungsmaschine ausgerichtet werden soll; wobei die zweite Bildgebungsmodalität ein Behandlungsmaschinen-Imager ist, um die Zielregion des Patienten bei der Behandlung und das Maschinenzentrum zu zeigen, wo es sich bekanntlich am Behandlungsmaschinencomputer für den Behandlungsstrahl an der Behandlungsmaschine befindet.

8. Verfahren nach Anspruch 1, wobei die erste Bildgebungsmodalität ein Planungsmaschinen-Imager ist, um eine Zielregion in einem Patienten (105) zur späteren Behandlung und das gewählte Behandlungsplanzentrum zu zeigen, wo der Behandlungsstrahl an der Behandlungsmaschine ausgerichet werden soll; wobei die zweite Bildgebungsmodalität mit einer Behandlungsmaschine gekoppelt ist, um die Zielregion des Patienten und das Maschinenzentrum der zweiten Bildgebungsmodalität zu zeigen, wo es sich bekanntlich am Behandlungsmaschinencomputer für den Behandlungsstrahl an der Behandlungsmaschine befindet.

9. Verfahren nach Anspruch 8, wobei der Versatz zwischen den ersten Koordinaten und den zweiten Koordinaten durch Ausrichten von wenigstens dem gewählten Behandlungsplanzentrum mit dem Maschinenzentrum ermittelt wird.

10. Verfahren nach Anspruch 8, wobei das Justieren des Behandlungsplans Folgendes beinhaltet:
Ermitteln einer Auflagejustierung zum Justieren einer den menschlichen Körper (1) tragenden Auflage auf der Basis der ermittelten Positionsänderung des Ziels relativ zum Maschinenzentrum und (2) relativ zu der Mehrzahl von Markern (110, 200); und
Bewegen der Auflage auf der Basis der Auflagenjustierung, um den ermittelten Versatz zu minimieren.

11. Verfahren nach Anspruch 10, wobei das Ermitteln des Versatzes das Berechnen einer relativen Position und einer relativen Orientierung von wenigstens einem aus der Mehrzahl von Markern (110, 200), die in der zweiten Modalität abgebildet wurden, relativ zu dem Maschinenzentrum beinhaltet, und
wobei das Ermitteln der Auflagejustierung das Benutzen der relativen Orientierung und der relativen Position der Marker relativ zum Maschinenzentrum beinhaltet.

12. Verfahren nach Anspruch 1, wobei das Ermitteln des Versatzes das Ausrichten des gewählten Behandlungsplanzentrums und des Maschinenzentrums zum Ausrichten eines geplanten Ziels mit dem Behandlungsmaschinenstrahl beinhaltet, auch wenn die Marker nicht ausgerichtet sind;
wobei der Versatz eine rotatorische Differenz im Markerversatz (815) identifiziert; und
wobei der Versatz eine x-, y-, z-Verschiebung im Markerversatz identifiziert.

## Revendications

1. Un procédé, comprenant :
la génération d'un premier ensemble de données d'image au moyen d'une première modalité d'imagerie, le premier ensemble de données d'image comprenant une première pluralité d'images d'un volume cible à l'intérieur d'un corps humain, et d'une pluralité de marqueurs (110, 200) implantés en interne à proximité ou par rapport au volume cible,
la génération d'un plan de traitement en fonction d'un centre de plan de traitement sélectionné et du premier ensemble de données d'image,
la détermination d'un premier système de coordonnées tridimensionnelles interne en fonction d'emplacements de la pluralité de marqueurs (110, 200) dans le premier ensemble de données d'image et le centre de plan de traitement sélectionné,
la détermination d'un premier ensemble de coordonnées tridimensionnelles d'emplacements de la pluralité de marqueurs dans le premier ensemble de données d'image en fonction du premier système de coordonnées interne,
la génération d'un deuxième ensemble de données d'image au moyen d'une deuxième modalité d'imagerie, le deuxième ensemble de données d'image comprenant une deuxième pluralité d'images du volume cible à l'intérieur du corps humain, et de la pluralité de marqueurs (110, 200) implantés en interne à proximité ou par rapport au volume cible,
la détermination d'un deuxième système de coordonnées tridimensionnelles interne en fonction d'emplacements de la pluralité de marqueurs (110, 200) dans le deuxième ensemble de données d'image et d'un isocentre de machine de traitement de la deuxième modalité d'imagerie,
la détermination d'un deuxième ensemble de coordonnées tridimensionnelles d'emplacements de la pluralité de marqueurs dans le deuxième ensemble de données d'image en fonction du deuxième système de coordonnées interne,
la détermination d'un décalage (815) entre le premier ensemble de coordonnées tridimensionnelles de la pluralité de marqueurs et le deuxième ensemble de coordonnées tridimensionnelles de la pluralité de marqueurs, et
l'ajustement du plan de traitement en fonction du décalage déterminé (815).

2. Le procédé selon la Revendication 1, où le premier ensemble de données d'image comprend une structure anatomique à proximité du volume cible, et comprend en outre :
la détermination d'une position de la structure anatomique par rapport au volume cible dans le deuxième ensemble de données d'image en fonction du deuxième ensemble de données d'image et du système de coordonnées interne.

3. Le procédé selon la Revendication 1, où la deuxième modalité d'imagerie est différente de la première modalité d'imagerie.

4. Le procédé selon la Revendication 1, où la deuxième modalité d'imagerie est identique à la première modalité d'imagerie.

5. Le procédé selon la Revendication 4, où la première modalité d'imagerie est une modalité d'imagerie à rayons x, la deuxième modalité d'imagerie est une modalité d'imagerie à rayons x couplée à une machine de traitement possédant la source de faisceau de traitement et le centre de machine d'un faisceau de rayonnement à haute énergie de la source de faisceau de la machine de traitement.

6. Le procédé selon la Revendication 1, où la première modalité d'imagerie est une imagerie CT et la deuxième modalité d'imagerie est une imagerie en kilovolt (kV), et où les marqueurs (110,200) sont imageables aux rayons x, internes et insérés dans un patient (105).

7. Le procédé selon la Revendication 1, où la première modalité d'imagerie est un imageur de machine de planification destiné à montrer le volume cible dans le corps humain pour un traitement ultérieur et le centre planifié de plan de traitement sélectionné où il est souhaité aligner le faisceau de traitement au niveau de la machine de traitement, où la deuxième modalité d'imagerie est un imageur de machine de traitement destiné à montrer la zone cible du patient au cours du traitement et à montrer le centre de machine où elle est connue se situer au niveau de l'ordinateur de la machine de traitement pour le faisceau de traitement au niveau de la machine de traitement.

8. Le procédé selon la Revendication 1, où la première modalité d'imagerie est un imageur de machine de planification destiné à montrer une zone cible chez un patient (105) pour un traitement ultérieur et le centre de plan de traitement sélectionné où il est souhaité aligner le faisceau de traitement au niveau de la machine de traitement, où la deuxième modalité d'imagerie est couplée à une machine de traitement destinée à montrer la zone cible du patient et à montrer le centre de machine de la deuxième modalité d'imagerie où elle est connue se situer au niveau de l'ordinateur de la machine de traitement pour le faisceau de traitement au niveau de la machine de traitement.

9. Le procédé selon la Revendication 8, où le décalage entre les premières coordonnées et les deuxièmes coordonnées est déterminé par l'alignement d'au moins le centre de plan de traitement sélectionné avec le centre de machine.

10. Le procédé selon la Revendication 8, où l'ajustement du plan de traitement comprend :
la détermination d'un ajustement de support de façon à ajuster un support soutenant le corps humain (1) en fonction du changement de position déterminé de la cible par rapport au centre de machine (2) et par rapport à la pluralité de marqueurs (110, 200), et
le déplacement du support en fonction de l'ajustement de support de façon à minimiser le décalage déterminé.

11. Le procédé selon la Revendication 10, où la détermination du décalage comprend le calcul d'une position relative et d'une orientation relative d'au moins un marqueur de la pluralité de marqueurs (110, 200) imagé dans la deuxième modalité, par rapport au centre de machine, et
où la détermination de l'ajustement de support comprend l'utilisation de l'orientation relative et de la position relative des marqueurs par rapport au centre de machine.

12. Le procédé selon la Revendication 1, où la détermination du décalage comprend l'alignement du centre de plan de traitement sélectionné et du centre de machine de façon à aligner une cible planifiée avec le faisceau de la machine de traitement, bien que les marqueurs ne soient pas alignés,
où le décalage identifie une différence rotationnelle dans le décalage de marqueur (815), et
où le décalage identifie un déplacement x, y, z dans le décalage de marqueur.
